# EUROPEAN PATENT APPLICATION

(11) **EP 4 257 692 A1**
(43) Date of publication of application: **11.10.2023**
(21) Application number: 21903818.9
(22) Date of filing: 07.12.2021
(51) Int. Cl.: C12N 15/70, C07K 14/435

(54) **RECOMBINANT MICROORGANISM HAVING IMPROVED ABILITY TO PRODUCE RECOMBINANT SILK PROTEIN AND METHOD FOR PRODUCING HIGH-MOLECULAR-WEIGHT RECOMBINANT SILK PROTEIN BY USING SAME**

(30) Priority: 07.12.2020 KR 20200169583
(71) Applicant: MEDICOSBIOTECH INC., Daejeon 34141 (KR)
(72) Inventor: LEE, Sang Yup, Daejeon 34141 (KR); KIM, Jiyong, Daejeon 34141 (KR)
(74) Representative: Mewburn Ellis LLP
(86) International application number: PCT/KR2021/018486
(87) International publication number: WO 2022/124772

(57) **Abstract**

The present invention relates to a recombinant microorganism having enhanced ability to produce recombinant silk protein and a method of producing high-molecular-weight recombinant silk protein using the same, and more specifically, to a recombinant microorganism having an enhanced ability to produce spider silk protein, obtained by introducing a sequence encoding a synthetic sRNA that inhibits expression of *eno* gene into a microorganism having ability to produce recombinant silk protein. The recombinant silk protein derived from the recombinant strain according to the invention has a high molecular weight similar to that of dragline silk protein produced by spiders, and may be mass-produced, and thus it has properties similar to or better than those of natural silk protein when manufactured into fibers. Accordingly, the recombinant silk protein may be advantageously used in various industrial fields such as bioengineering and/or pharmaceutical fields where spider silk fibers are expected to be applied.

## Description

### Technical Field

The present invention relates to a recombinant microorganism having an enhanced ability to produce recombinant silk protein and a method of producing high-molecular-weight recombinant silk protein using the same, and more specifically, to a recombinant microorganism having an enhanced ability to produce spider silk protein, obtained by inhibiting expression of *eno* gene in a microorganism having the ability to produce recombinant silk protein.

### Background Art

Natural silk protein materials have been used as representative clothing materials for a long period of time. Recently, they have been highly likely to be used as next-generation highly functional industrial materials because natural silk proteins have excellent biocompatibility, excellent elasticity and physical strength, can be shaped in various forms such as powder, membranes, porous bodies, and gels, and can be chemically modified.

Until now, it has been known that silk proteins are mainly produced by silkworms and spiders.

Since silkworms can be farmed and silkworm silk proteins can be produced in an amount of up to 5 kg from about 1.5 million silkworms in an area of 300 m², silkworm silk proteins have traditionally been widely used in textile manufacturing. In addition, natural silkworm silk proteins are human-friendly materials because they cause no immune or allergic reactions. It has been suggested that natural silkworm silk proteins can be used as industrial materials such as cosmetic materials, enzyme immobilization supports, cell culture materials, and artificial skin. However, silkworm silk proteins lack physical properties in terms of firmness, that is, strength, and thus they are still somewhat restricted for use as industrial materials, and currently, various studies are being conducted to improve the physical properties of silkworm silk proteins. For example, these studies include studies on the molecular weight-dependent separation and functionality of silk fibroin, which is the main component of silkworm silk protein, studies on the preparation of silkworm silk protein fine powder and skin compatibility thereof, studies on improving the physical properties of silkworm silk protein by chemically synthesized polymers, and studies on mixing of silkworm silk protein with natural sugar-based polymer compounds.

In the case of spider silk proteins, since it was recently found that the physical properties of spider silk proteins obtained from nature are excellent in terms of firmness and elasticity, studies on the use of spider silk proteins as industrial materials have been actively conducted. However, it is known that it is impossible to farm spiders due to their cannibalistic nature, and it is practically impossible to isolate, produce and use natural proteins, like silkworm silk proteins, because the amount of silk proteins produced by spiders is very small. Therefore, studies related to spider silk proteins are mainly focused on producing recombinant proteins by introducing spider silk protein genes into various protein expression systems, and various attempts have been made to mass-produce spider silk proteins in *E. coli,* yeast, plant cells, animal cells, transgenic animals, or the like. In general, regarding studies related to spider silk proteins, some outstanding research results have recently been reported due to intensive research and investment, mainly since the 2000s, but unique characteristics such as the repetitive DNA sequence of the spider silk protein gene and the repetition of specific amino acid sequences such as glycine, alanine, and serine limit the recombinant production of silk protein materials that exist in nature.

Dragline spider silk, which is used as the spider's lifeline and is used to make radial spider webs, has high strength and elasticity. Spider silk is five times stronger per unit mass than steel and three times harder than man-made high-quality Kevlar fiber (Gosline, J. M. et al., J. Exp. Biol., 202:3295, 1999; Vollrath, F. & Knight, D. P., Nature 410: 541, 2001). In addition, since spider silk has biodegradable properties, it can be applied to biomedical fields such as sutures and scaffolds, and since it has an anti-inflammatory function, it can be applied as an antibacterial material. In addition, spider silk can also be used in a variety of cosmetic materials because of its excellent moisturizing power. Therefore, dragline spider silk has received a lot of attention as a material with various industrial applications.

Unfortunately, however, natural dragline silk cannot be easily obtained by culturing spiders, because spiders have strong territorial instincts and are aggressive. Therefore, numerous efforts have been made to produce recombinant dragline silk proteins (Lazaris, A. et al., Science, 295:472, 2002; Teule, F. et al., Nat. Protoc., 4: 341, 2009; Arcidiacono, S. et al., Macromolecules, 35: 1262, 2002; Brooks, A. E. et al. Biomacromolecules, 9: 1506, 2008; Heim, M., Keerl, D. & Scheibel, T., Angew. Chem. Int. Ed. Engl., 48: 3584, 2009; Fahnestock, S. R. et al., Rev. Mol. Biotechnol., 74: 105, 2000; Scheller, J. et al., Nat. Biotechnol., 19: 573, 2001; Widmaier, D. M. et al. Mol. Syst. Biol., 5: 309, 2009).

All spiders studied to date have naturally produced dragline silk proteins having a high molecular weight of 250 to 320 kDa. Regarding dragline silk proteins synthesized in *E. coli*, there have been reports on the production of a dragline silk protein having a molecular weight of 163 kDa (Fahnestock S.R. & Irwin S.L., Appl. Microbiol. Biotechnol., 47:23, 1997), a dragline silk protein having a molecular weight of 284.9 kDa (KR 10-1317420; Xia, Xiao-Xia, et al. Proceedings of the National Academy of Sciences, Vo.107(32), pp. 14059-44063, 2010), a dragline silk protein having a molecular weight of 386.8 kDa using rpnA gene expression inhibition (KR 10-101765255), and a dragline silk protein having a molecular weight of 556 kDa using split intein-mediated ligation (Christopher H. Bowen et al., Biomacromolecules, Vol. 19, 9, pp. 3853-3860, 2018). However, there is a problem in that both protein yield and good quality thread-assembly sufficient for commercial use are not achieved.

Therefore, there is a need for a strain capable of producing a high-molecular-weight recombinant silk protein with high purity.

Accordingly, the present inventors have made extensive efforts to solve the above-described problems and to develop a strain capable of mass-producing a high-molecular-weight recombinant silk protein with high purity, and as a result, have found that, when the expression of genes identified as a result of MOMA simulation is inhibited using sRNA, it is possible to mass-produce a high-molecular-weight recombinant silk protein, thereby completing the present invention.

### Summary of the Invention

An object of the present invention is to provide a recombinant strain capable of producing a high-molecular-weight recombinant silk protein.

Another object of the present invention is to provide a method of producing a high-purity, high-molecular-weight recombinant silk protein using the strain.

In order to achieve the above objects, the present invention provides a recombinant microorganism having an enhanced ability to produce spider silk protein, obtained by inhibiting expression of *eno gene* in a microorganism having an ability to produce recombinant silk protein.

The present invention also provides a method for producing recombinant silk protein, the method comprising steps of: (a) producing recombinant silk protein by culturing the recombinant microorganism; and (b) recovering the produced recombinant silk protein.

### Brief Description of Drawings

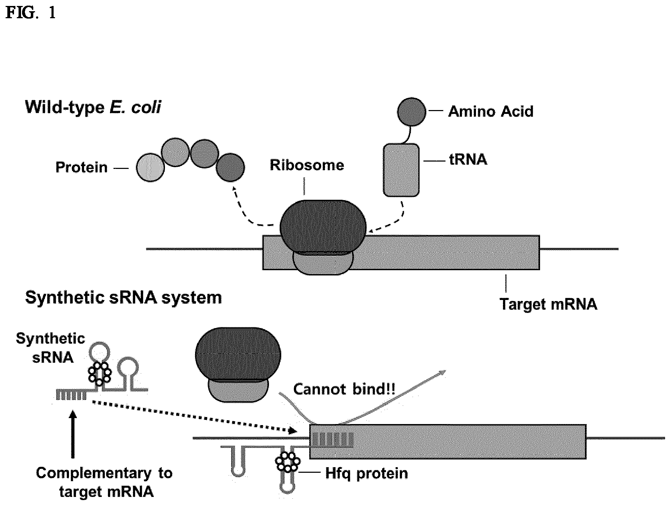

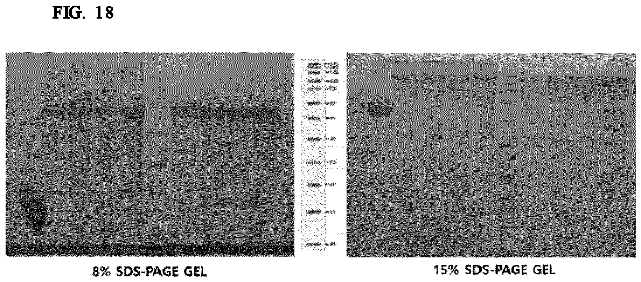

FIG. 1 is a conceptual view explaining the mechanism of action of a synthetic sRNA of the present invention.
FIG. 2(A) is a conceptual view showing an sRNA cassette for the *eno* gene screened according to one example of the present invention, and FIG. 2(B) is a map of a vector comprising the cassette.

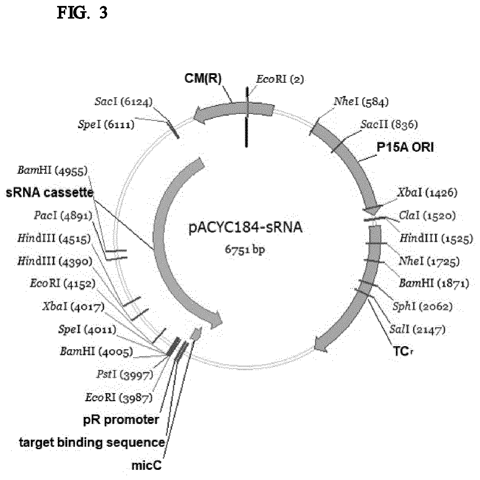

FIG. 3 is a map of a vector comprising an sRNA cassette for an additional gene screened according to one example of the present invention.
FIG. 4(A) shows the results of analyzing the expression level of spider silk protein depending on the length of the target binding site of the *eno* gene screened according to one example of the present invention, FIG. 4(B) shows the results of calculating binding energy, and FIG. 4(C) is a graph showing the relationship between binding energy and expression level.
FIGS. 5(A) and 5(B) show the results of analyzing the expression levels of recombinant silk protein in known strains, and FIG. 5(C) shows the results of comparing the expression level of recombinant silk protein following the inhibition of expression of the *eno* gene screened according to one example of the present invention.
FIG. 6 shows the result of analyzing the expression levels of spider silk protein following knockdown of additional candidate genes screened according to one example of the present invention.
FIG. 7 shows the result of analyzing the expression levels of spider silk protein following knockdown of additional candidate genes screened according to one example of the present invention.
FIG. 8 shows the result of analyzing the expression levels of spider silk protein following knockdown of additional candidate genes screened according to one example of the present invention.
FIG. 9(A) shows the results of analyzing the expression levels of spider silk protein following knockdown of the first screened genes among the additional candidate genes screened according to one example of the present invention, and FIG. 9(B) is a graph quantifying the results.
FIG. 10(A) shows the results of analyzing the expression level of spider silk protein depending on the length of the target binding site of the *ychM* gene screened according to one example of the present invention, FIG. 10(B) shows the results of calculating binding energy, and FIG. 10(C) is a graph showing the relationship between binding energy and expression level.

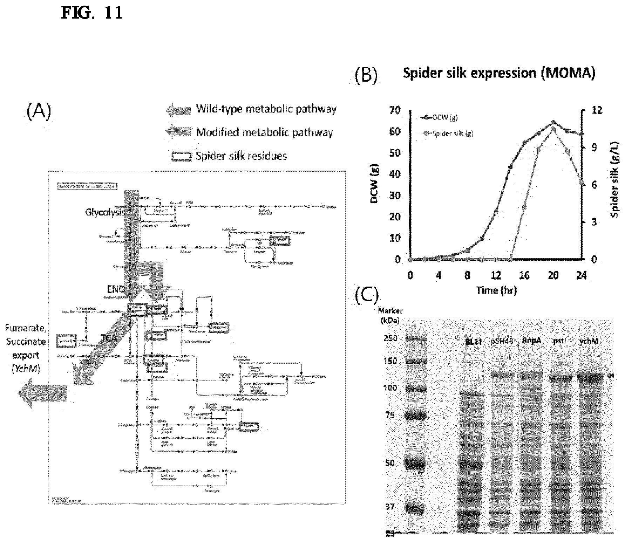

FIG. 11(A) is a schematic view predicting a modified metabolic pathway in a strain in which the expression of the *eno* gene and *ychM* gene screened according to one example of the present invention has been inhibited, FIG. 11(B) shows the results of measuring the expression level of recombinant silk protein in a strain, in which the expression of the *eno* gene and *ychM* gene screened according to one example of the present invention has been inhibited, 6 hours after induction with 1 mM IPTG when the OD₆₀₀ at 30°C reached 100 during fed-batch fermentation of the strain, and FIG. 11(C) shows the results of analyzing the expression level of the recombinant silk protein produced in a strain in which the expression of additional candidate genes in addition to the expression of the *eno* gene has been inhibited.
FIG. 12 shows the results of RNA-seq analysis of strains in which the expression of the *eno* gene and *ychM* gene screened according to one example of the present invention has been inhibited. The left panel is a list of the most up-regulated genes, and the right panel is a list of the most down-regulated genes.
FIG. 13 is a heatmap of a DEG list obtained using RNA-seq of strains in which the expression of the *eno* gene and *ychM* gene screened according to one example of the present invention has been inhibited, and shows that various genotypes were changed compared to those in existing recombinant silk protein strains.
FIG. 14(A) the result of confirming the expression changes of glycine-, alanine-, and serine-related genes in strains in which the expression of the *eno* gene and *ychM* gene screened according to one example of the present invention has been inhibited, FIG. 14(B) shows the results of confirming the expression changes of genes related to transcription and translation, and FIG. 14(C) shows the results of confirming the amplification of glycine, alanine and serine tRNA.
FIG. 15 shows the results of optimizing the concentration of ammonium persulfate in the salting-out method for isolation and purification of a strain in which the expression of the *eno* gene and *ychM* gene screened according to one example of the present invention has been inhibited.
The left panel of FIG. 16 shows the results of analyzing the purity of the recombinant silk protein isolated and purified before microfiltration, and the right panel shows the results of analyzing the purity after microfiltration.

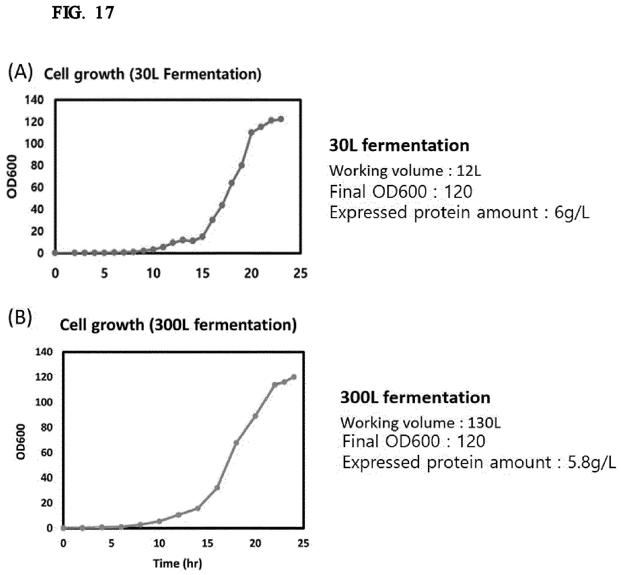

FIG. 17(A) shows the results of 30-L fermentation of a strain in which the expression of the *eno* gene and *ychM* gene screened according to one example of the present invention has been inhibited, and FIG. 17(B) shows the results of 300-L fermentation of the strain.
The left panel of FIG. 18 shows the results of 8% SDS-PAGE of the isolated and purified recombinant protein after 30-L fermentation of the strain according to one example of the present invention, and the right panel shows the results of 15% SDS-PAGE of the isolated and purified recombinant protein.

### Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification and the experimental methods described below are well known and commonly used in the art.

The definition of main terms used in the detailed description of the invention is as follows

As used herein, the term "silk protein" refers to a synthetic silk protein that approximates the molecular and structural profile of native silk proteins and is biosynthesized by a recombinant protein production method. Examples of the silk protein include dragline silk, silk fibroin, and flagelliform silk proteins. As used herein, the term "silk-like protein" refers to a protein which comprises, as a repeat unit, a peptide having a glycine content of 10% or more, similar to the silk protein, and is produced by, for example, a recombinant protein production method. Examples of the silk-like protein include elastin, byssus, and collagen.

As used herein, the term "spider silk fiber" refers to a fiber which is produced from a synthetic recombinant silk protein and is substantially similar to a native spider silk fiber. The term "spider silk-like fiber" refers to a fiber which is produced from a synthetic recombinant silk-like protein and has physical properties similar to those of a spider silk protein.

As used herein, the term "recombinant protein" refers to a protein produced by expression of a nucleic acid sequence which is incorporated into a vector, i.e., e.g., an autonomously replicating plasmid or virus, or into the genomic DNA of a host cell, or which exists as a separate molecule in the host cell.

As used herein, the term "host cell" refers to any cell capable of expressing a functional gene and/or gene product introduced from another cell or organism.

As used herein, the term "sRNA (small RNA)" is a short RNA having a nucleotide sequence length of 200 or fewer nucleotides, which is not translated into protein and effectively inhibits the translation of a specific mRNA by complementary binding.

As used herein, the term "ribosome binding site" refers to an mRNA site to which a ribosome binds for mRNA transcription.

As used herein, "gene" should be regarded in the broadest sense, and may encode a structural protein or a regulatory protein. Here, the regulatory protein includes a transcription factor, a heat shock protein, or a protein involved in DNA/RNA replication, transcription, and/or translation. In the present invention, the target gene whose expression is to be inhibited may exist as an extrachromosomal element.

In the present invention, in order to develop a strain capable of mass-producing a high-molecular-weight recombinant silk protein by manipulating metabolic pathways in an existing strain producing a high-molecular-weight recombinant silk protein, candidate genes were screened using an sRNA library capable of inhibiting expression of each gene, and then an optimal gene combination was screened by comparing the production of recombinant silk protein between the screened genes. In this case, it was confirmed that it was possible to produce a high-molecular-weight recombinant silk protein with high purity.

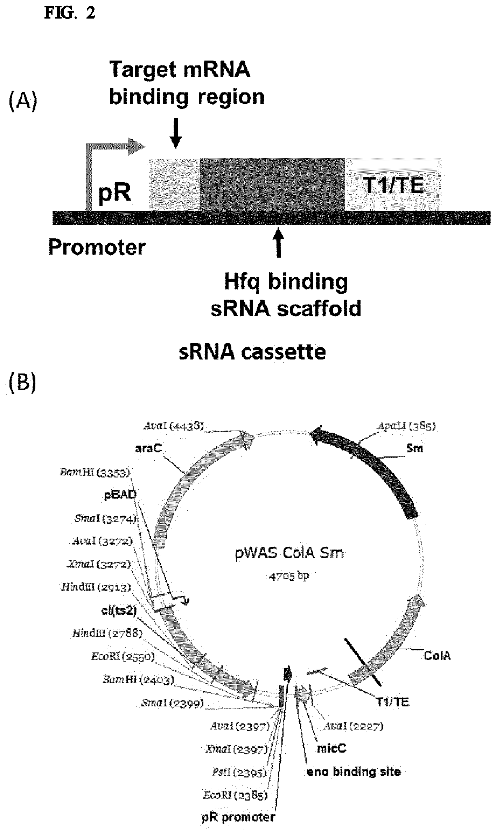

That is, in one example of the present invention, an sRNA library capable of knocking down each of all *E*. *coli* genes was introduced into an existing strain producing recombinant silk protein (KR 10-1317420), and then candidate genes whose inhibition of expression was expected to increase the production of recombinant silk protein were screened through a metabolic pathway simulation using knock-down regulation of two or more genes. It was confirmed that, when expression of the genes was inhibited using sRNA, the production of recombinant silk protein increased (FIGS. 2, 4 and 5).

Additionally, other candidate genes were tested in the same manner, and it was confirmed that, when expression of additional genes in addition to the genes screened in the above step, the production of recombinant silk protein dramatically increased (FIG. 11).

Therefore, in one aspect,
the present invention is directed to a recombinant microorganism having an enhanced ability to produce spider silk protein, obtained by inhibiting expression of *eno* gene in a microorganism having the ability to produce recombinant silk protein.

In the present invention, the inhibition of expression of the *eno* gene may be performed using any method known to those skilled in the art, and preferably, may be performed by at least one method selected from the group consisting of a method of introducing a sequence encoding a synthetic sRNA that inhibits expression of the gene, a method of modifying the promoter, a method of introducing antisense RNA, and a method of introducing siRNA, without being limited thereto.

In addition, the recombinant microorganism may be one in which expression of at least one additional gene selected from the group consisting of *gnd, ybcF, araC, purT, tdcD, ackA, pta, fsaA, fsaB, ptsH, ptsI, dhal, dhaK, pyrD, guaA, prsA, tesB, aas, acpP, ychM, cysZ, asnA, asnB, acpP, plsB, fadB, sucC, fldA, fade, sucA, sucB,* and *pykA* has been inhibited.

In the present invention, the additional gene may be *pstI, asnA, ychM, tesB, dhaK, guaA, pck,* or *purT,* more preferably *ychM* or *pstI.*

In the present invention, the inhibition of expression of the *gnd, ybcF, araC, purT, tdcD, ackA, pta, fsaA, fsaB, ptsH, ptsI, dhal, dhaK, pyrD, guaA, prsA, tesB, aas, acpP, ychM, cysZ, asnA, asnB, acpP, plsB, fadB, sucC, fldA, fade, sucA, sucB,* or *pykA* gene may performed using any method known to those skilled in the art, and preferably, may be performed by at least one method selected from the group consisting of a method of introducing a sequence encoding a synthetic sRNA that inhibits expression of the gene, a method of modifying the promoter, a method of introducing antisense RNA, and a method of introducing siRNA, without being limited thereto.

In the present invention, information on the gene may be gene information described in https://www.uniprot.org/, without being limited thereto.

In the present invention, the microorganism having the ability to produce recombinant silk protein may be one into which a gene encoding a recombinant silk protein in which a peptide having the amino acid sequence of SEQ ID NO: 1 is repeated 1 to 160 times, preferably 8 to 160 times, more preferably 16 to 160 times, or a recombinant vector containing the gene, and a recombinant vector containing a nucleotide sequence encoding tRNA, have been introduced.

In the present invention, the microorganism having the ability to produce recombinant silk protein may be *E. coli* BL21 (DE3) ((F- ompT hsdSB(rB- mB-) gal dcm (DE3) pTet-glyVXY(CmR) pSH16a, 32, 64, 96(KanR)), without being limited thereto.

In the present invention, the nucleotide sequence encoding tRNA may be a nucleotide sequence encoding glycine tRNA.

In the present invention, the peptide may be a repeating peptide unit constituting a dragline silk protein.

In the present invention, the peptide having a glycine content of 10% or more, which constituents the silk protein or silk-like protein, may be a repeating peptide unit constituting a protein selected from the group consisting of dragline silk, elastin, silk fibroin, byssus, flagelliform silk, and collagen. The amino acid sequences of SEQ ID NOs: 1 to 4 are repeating peptide units of dragline silk protein, the amino acid sequences of SEQ ID NOs: 5 to 7 are repeating peptide units of elastin, the amino acid sequence of SEQ ID NO: 8 is a repeating peptide unit of silk fibroin, the amino acid sequence of SEQ ID NO: 9 is a repeating peptide unit of byssus, the amino acid sequence of SEQ ID NO: 10 is a repeating peptide unit of flagelliform silk, and SEQ ID NOs: 11 and 12 are repeating peptide units of collagen. 
SEQ ID NO 1: NH2-
SGRGGLGGQGAGMAAAAAMGGAGQGGYGGLGSQGT-COOH
SEQ ID NO 2: NH₂-GPGQQ-COOH
SEQ ID NO 3: NH₂-GPGGY-COOH
SEQ ID NO 4: NH₂-GGYGPGS-COOH
SEQ ID NO 5: NH₂-GVGVP-COOH
SEQ ID NO 6: NH₂-VPGG-COOH
SEQ ID NO 7: NH₂-APGVGV-COOH
SEQ ID NO 8: NH₂-GAGAGS-COOH
SEQ ID NO 9: NH₂-GPGGG-COOH
SEQ ID NO 10: NH₂-GPGGX-COOH
SEQ ID NO 11: NH₂-GAPGAPGSQGAPGLQ-COOH
SEQ ID NO 12: NH₂-GAPGTPGPQGLPGSP-COOH

In the present invention, it was confirmed that a recombinant silk protein prepared to comprise 48 repeats of the amino acid sequence set forth in SEQ ID NO: 1 (hereinafter referred to as "48-mer") had a molecular weight of about 148 kDa, and the production thereof was significantly higher than the production of recombinant silk protein in an existing silk. In addition, it was found that a recombinant silk protein prepared to comprise 96 repeats of the amino acid sequence set forth in SEQ ID NO: 1 (hereinafter referred to as "96-mer") had a molecular weight reaching 284.9 kDa, which is substantially similar to the molecular weight of native silk protein (250 to 320 kDa) obtained from spiders, and the production and purity thereof significantly increased compared to those in an existing strain.

However, recombinant silk proteins comprising 160 repeats or more of the peptide sequence cannot be synthesized by *E. coli.* Thus, the recombinant silk or silk-like protein according to the present invention may have a structure in which the peptide is repeated 16 to 160 times, preferably 48 to 160 times, more preferably 96 to 160 times.

In the present invention, the amino acid sequences that are used as repeat units are not limited to the exact sequences of SEQ ID NOS: 1 to 12. The amino acid sequences herein also comprise variants. Thus, the amino acid sequences of the proteins of the present invention also encompass all sequences differing from the herein-disclosed sequences by amino acid insertions, deletions, and substitutions.

Preferably, amino acid "substitutions" are the result of replacing one amino acid with another amino acid having similar structural and/or chemical properties, i.e., conservative amino acid replacements. Amino acid substitutions may be made on the basis of similarity in the polarity, charge, solubility, hydrophobicity, hydrophilicity, and/or amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" in the repeat unit are typically in the range of about 1 to 5 amino acids, and preferably about 1, 2 or 3 amino acids. Amino acid additions in the repeat unit are typically less than 100, preferably less than 80, more preferably less than 50, most preferably less than 20 amino acids, which are inserted into the repeat unit of the present invention and added to and/or inserted into the protein of the present invention. It is noted that only those additions, which do not negatively affect the required characteristics of the protein disclosed herein, are contemplated in the present invention.

The variation allowed may be experimentally determined by systematically making insertions, deletions, or substitutions of amino acids in a protein using recombinant DNA techniques and assaying the resulting recombinant variants for activity. This does not require more than routine experiments for a person skilled in the art.

Accordingly, the protein of the present invention may comprise, as a repeat unit, an amino acid sequence having a homology of at least 90% to SEQ ID NO: 1. As used herein, the phrase "having a homology of at least 90%" means that the protein has an identity of 91, 91.5, 92, 92.5, 93, 93.5, 94, 94.5, 95, 95.5, 96, 96.5, 97, 97.5, 98, 98.5, 99 or 99.5% with the sequence of SEQ ID NO: 1. The term "homology" refers to the degree of similarity between two amino acid sequences. Homologous proteins are those that are similar in sequence and function. Homology comparison may be conducted by eye, but usually, may be performed by calculating the percent homology between two or more sequences with the aid of readily available sequence comparison programs (Wilbur, W. J. & Lipman, D. J., Proc. Natl. Acad. Sd. USA., 80:726, 1983) .

In the present invention, the synthetic sRNA may comprise: an Hfq binding site derived from sRNA of any one of MicC, SgrS and MicF; and a region forming a complementary bond with the mRNA of the target gene.

In the present invention, the sequence encoding the synthetic sRNA that inhibits expression of the *eno* gene may be represented by the nucleotide sequence of SEQ ID NO: 16.
SEQ ID NO: 16 (eno): ATGTCCAAAATCGTAAAA

In the present invention, the sequences encoding the synthetic sRNAs that inhibit expression of the *pstI, asnA, ychM, tesB, dhaK, guaA, pck,* and *purT* gene may be represented by the nucleotide sequences of SEQ ID NOs: 17 to 24, respectively.
SEQ ID NO: 17 (pstI): ATGATTTCAGGCATTTTAGCATCC
SEQ ID NO: 18 (asnA): ATGAAAACCGCTTACATTGCCAAA
SEQ ID NO: 19 (ychM): GTGAACAAAATATTTTCCTC
SEQ ID NO: 20 (tesB): ATGAGTCAGGCGCTAAAA_AATTTA
SEQ ID NO: 21 (dhaK): ATGAAAAAATTGATCAATGATGTG
SEQ ID NO: 22 (guaA): ATGACGGAAAACATTCATAAGCAT
SEQ ID NO: 23 (pck): ATGCGCGTTAACAATGGTTTGACC
SEQ ID NO: 24 (purT): ATGACGTTATTAGGCACTGCGCTG

In the present invention, as a method of introducing the sequence encoding the synthetic sRNA that inhibits expression of the target gene into the microorganism, any method known to those skilled in the art may be used. Preferably, a recombinant vector containing the sequence encoding the synthetic sRNA that inhibits expression of the target gene may be used, or a method of introducing the sequence encoding the synthetic sRNA that inhibits expression of the target gene directly into the chromosome of a microorganism may be used, without being limited thereto.

In the present invention, a recombinant vector containing the sequence encoding the synthetic sRNA that inhibits expression of the target gene may contain: a promoter; an Hfq binding site derived from sRNA of any one of MicC, SgrS and MicF; a region forming a complementary bond with a target gene mRNA; and a terminator.

In the present invention, the promoter may be any type of promoter capable of inducing expression of sRNA, is preferably selected from the group consisting of tac, trc, T7, BAD, APR, and Anderson synthetic promoters, and may be represented by the nucleotide sequence of SEQ ID NO: 13.

SEQ ID NO 13: 5′-
taacaccgtgcgtgttgactattttacctctggcggtgataatggttgc-3′

In the present invention, the Hfq binding site may preferably be derived from MicC and may be represented by the nucleotide sequence of SEQ ID NO: 14.

SEQ ID NO 14:
5′-
tttctgttgggccattgcattgccactgattttccaacatataaaaagacaagcccg
aacagtcgtccgggcttttttt-3′

In the present invention, the terminator may be any type of terminator capable of terminating transcription of sRNA, is preferably a T1/TE terminator, and is most preferably represented by the nucleotide sequence of SEQ ID NO: 15.

SEQ ID NO: 15:

5'-

ccaggcatcaaataaaacgaaaggctcagtcgaaagactgggcctttcgttttatct

gttgtttgtcggtgaacgctctctactagagtcacactggctcaccttcgggtgggc

ctttctgcgtttata-3'

As used herein, the term "complementary bond" refers to base pairing between nucleic acid sequences, and means that the sequence of a partial region of a target gene mRNA and the sequence of the region forming a complementary bond with the target gene mRNA are complementary to each other by about 70 to 80% or more, preferably about 80 to 90% or more, even more preferably about 95 to 99% or more.

As used herein, the term "vector" means a DNA construct containing a DNA sequence operably linked to a suitable control sequence capable of expressing DNA in a suitable host. The vector may be a plasmid, a phage particle, or simply a potential genome insert. Once transformed into a suitable host, the vector may replicate and function independently of the host genome, or may, in some instances, integrate into the genome itself. Since a plasmid is the most commonly used type of vector, the terms "plasmid" and "vector" are sometimes used interchangeably throughout the specification of the present invention. For the purpose of the present invention, a plasmid vector is preferably used. A typical plasmid vector that may be used for this purpose contains: (a) a replication origin by which replication occurs efficiently such that several to several hundred plasmid vectors per microorganism are created; (b) an antibioticresistant gene by which microorganisms transformed with the plasmid vector can be selected; and (c) restriction enzyme cutting sites into which foreign DNA fragments can be inserted. Even if suitable restriction enzyme cutting sites are not present in the vector, the use of a conventional synthetic oligonucleotide adaptor or linker enables easy ligation between the vector and the foreign DNA fragments. After ligation, the vector should be transformed into a suitable microorganism. The transformation can be easily achieved by the calcium chloride method or electroporation (Neumann, et al. EMBO J., 1:841, 1982). As the vector used for the expression of sRNA according to the present invention, any expression vector known in the art may be used.

A nucleotide sequence is operably linked when it is arranged in a functional relationship with another nucleic acid sequence. The nucleotide sequence may be a gene and control sequence(s) linked to be capable of expressing the gene when a suitable molecule (e.g., transcription-activating protein) binds to the control sequence (s). For example, DNA for a pre-sequence or a secretory leader is operably linked to DNA encoding polypeptide when expressed as pre-protein participating in secretion of polypeptide; a promoter or an enhancer is operably linked to a coding sequence when affecting the transcription of the sequence; or a ribosome binding site is operably linked to a coding sequence when arranged to facilitate translation. Generally, the term "operably linked" means that the linked DNA sequences are contiguous, and in the case of the secretory leader, are contiguous and present in a reading frame. However, an enhancer is not necessarily contiguous. However, an enhancer is not necessarily contiguous. The linkage between these sequences is performed by ligation at a convenient restriction enzyme site. However, when the site does not exist, a synthetic oligonucleotide adaptor or a linker is used according to a conventional method.

In the present invention, the microorganism may be selected from the group consisting of *E*. *coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium.*

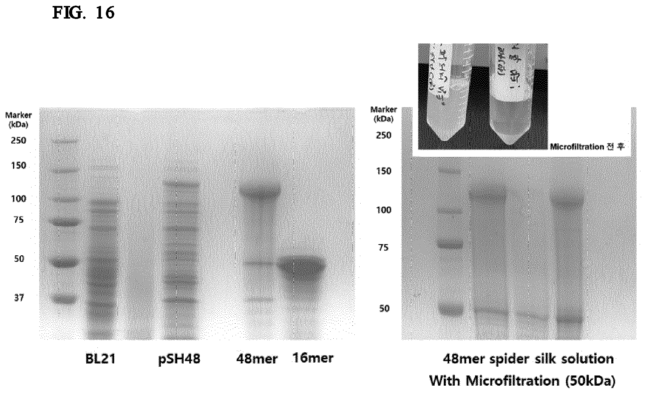

Meanwhile, in another example of the present invention, it was confirmed that, when recombinant silk protein was produced using the strain and then isolated and purified using a salting-out method, a high-purity recombinant silk protein could be obtained (FIG. 16).

Therefore, in another aspect, the present invention is directed to a method for producing recombinant silk protein, the method comprising steps of:
(a) producing recombinant silk protein by culturing the recombinant microorganism; and (b) recovering the produced recombinant silk protein.

In the present invention, step (b) may comprise a salting-out step.

Preferably, the step of recovering the recombinant silk protein may be performed by the following steps, without being limited thereto.
Step (a) of settling down the cells of the fermentation broth (4,000 rpm/30 min), and then resuspending the cells in buffer A (1mM Tris-HCl (pH8.0), 20 mM NaH₂PO₄) solution containing 8M urea and 2M thiourea;
step (b) of mixing the cell suspension using a magnet at 25°C for 6 hours;
step (c) of pH to 4.0 using glacial acetic acid, followed by mixing using a magnet at 25°C for 2 hours;
step (d) of centrifuging at 10,000 rpm for 20 minutes;
step (e) of collecting the supernatant, adding (NH₄)₂SO₄ thereto to a concentration of 0.85 M, followed by precipitation by mixing for 6 hours;
step (f) of centrifuging at 100,000 rpm for 20 minutes;
step (g) of collecting the supernatant, followed by precipitation using 1.3M (NH₄)₂SO₄;
step (h) of collecting the precipitate, followed by dissolution using a solution containing buffer A and 8M urea;
step (i) of performing dialysis using a solution containing 1.5M urea and buffer A for 12 hours;
step (j) of performing dialysis using buffer A for 12 hours; and
step (k) of freeze-drying the pellet collected by centrifugation.

In the present invention, the centrifuged pellet may be concentrated by various known methods and then freeze-dried.

### Examples

Hereinafter, the present invention will be described in more detail with reference to examples. These examples are only for illustrating the present invention, and it will be apparent to those skilled in the art that the scope of the present invention is not to be construed as being limited by these examples.

### Example 1. Construction and introduction of sRNA suitable for recombinant silk protein-producing strain

An sRNA system (Na, Dokyun et al., Nature biotechnology, 31.2, pp. 170-174, 2013) constructed so as to be able to knock down each *E. coli* gene individually was newly introduced into a known recombinant silk protein-producing strain (KR 10-1317420) depending on a spider silk protein-producing strain. A new sRNA platform was constructed using a pColA-Sm vector having streptomycin (hereinafter referred to as Sm) as a marker and a pACYC184-Cm vector having chloramphenicol (hereinafter referred to as Cm) as a marker so as not to overlap the marker kanamycin (referred to as Km) of a spider silk protein-producing vector and the origin of replication ColE.

The synthetic regulatory sRNA used in the present invention consisted of PR promoter, MicC scaffold and T1/TE terminator. The two synthetic regulatory sRNA platform plasmids were constructed by Gibson assembly of three DNA fragments, including an sRNA fragment, an antibiotic marker (antibiotic resistance gene) fragment, and a replication origin fragment (D.G. Gibson et al., Nature Methods (2009), 6(5), 343-345).

### Example 2. Target gene selection

To screen effective gene deletion targets for increased production of spider silk protein, MOMA (minimization of metabolic adjustment) simulation was introduced. For MOMA simulation, in order to construct a spider silk protein production metabolic network model using gene-protein-biochemical reaction relationship, a microbial metabolic network model was constructed using the glycine and serine production pathways that form the core of the spider silk protein structure. Metabolic characteristics such as metabolic flux were analyzed using the constructed model, and deletion-target pairs that increase the production of spider silk protein when knocked down were screened using a simulation based on metabolic flux analysis.

The deletion target screening method according to the present invention was performed by introducing a quadratic programming method, unlike an existing linear method (Segre et al. Proc. Nat.l Acad. Sci. 99: 15112-15117. 2002). Therefore, deletion targets can be efficiently predicted based on the simultaneous effect of knockdown of various targets rather than a single target on the expression of a target product.

As a result, as shown in Table 1 below, about 30 gene pairs were selected from about 20 reaction pairs predicted to be the most effective targets, and the *eno* gene was present commonly in all of about 30 corresponding gene pairs. Thus, the *eno* gene was first knocked down, followed by optimization.

**[Table 1] Table. Overall results of MOMA simulation**

| **Target1** | **Target2** | **Corresponding genes** | **Biomass flux** | **Production flux** |
|---|---|---|---|---|
| ENO | GND | *gnd* | 0.396543 | 0.061807 |
| ENO | CBMKr | *ybcF, arcC* | 0.372821 | 0.057653 |
| ENO | ACKr | *purT, tdcD, ackA* | 0.378326 | 0.057237 |
| ENO | PTAr | *pta* | 0.378326 | 0.057237 |
| ENO | F6PA | *fsaA, fsaB* | 0.38008 | 0.057104 |
| ENO | DHAPT | *ptsH, ptsI, dhaL, dhaK* | 0.38008 | 0.057104 |
| ENO | DHORD2 | *pyrD* | 0.381865 | 0056969 |
| ENO | GMPS | *guaA* | 0.382662 | 0056908 |
| ENO | PRPPS | *prsA* | 0.388862 | 0.056875 |
| ENO | FACOAE161 | *tesB* | 0.385485 | 0.056853 |
| ENO | AACPS4 | *aas*, *acpP* | 0.385485 | 0.056853 |
| ENO | SO4t2pp | *ychM, cysZ* | 0.383507 | 0.056844 |
| ENO | ASNS2 | *asnA, asnB* | 0.384019 | 0056806 |
| ENO | G3PAT161 | *acpP, plsB* | 0.386626 | 0.056798 |
| ENO | CTECOAI7 | *fadB* | 0.383772 | 0.056792 |
| ENO | SUCOAS | *sucC* | 0.384936 | 0.056785 |
| ENO | POR5 | *fldA* | 0.384363 | 0.05678 |
| ENO | ACOAD7f | *fadE* | 0.38394 | 0.05678 |
| ENO | AKGDH | *sucA, sucB* | 0.384941 | 0.056773 |
| ENO | PYK2 | *pykA* | 0.384474 | 0.056771 |

### Example 3. Verification of effect of knockdown of first selected eno gene

Based on the pColA-Sm vector, a synthetic regulatory sRNA for knocking down the *eno* gene was constructed using the pR promoter, MicC scaffold, and T1/TE terminator (Table 3). Since the *eno* gene is an essential gene for the growth of *E. coli*, it interferes with cell growth when completely knocked out or knocked down with high efficiency. Therefore, the length of the target binding site, which is the gene site to which sRNA binds, was adjusted to 15-nt to 24-nt (Table 2) to obtain the optimal knockdown effect.

At this time, to calculate binding energy, the UNAFOLD (Unified Nucleic Acid Folding) program, which calculates the binding energy depending on the length and sequence information of nucleotides, was used (Markham, Nicholas R., and Michael Zuker. Bioinformatics. Humana Press, 2008).

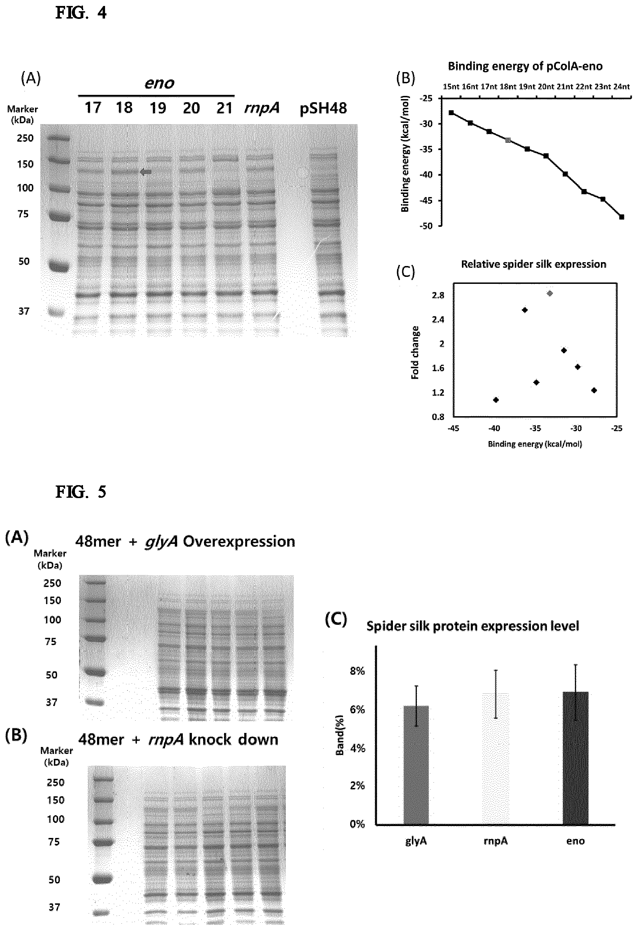

As a result, as shown in FIG. 4, it could be confirmed that, as the length of the binding site increased, the binding energy decreased and the knockdown effect increased. In addition, through experiments, it could be confirmed that the knockdown effect was poor at a binding site having a length of 15-nt or 16-nt, and when a binding site having a length of 22-nt or more was used, cell growth was greatly inhibited due to an excessively high knockdown effect. Finally, as a result of analyzing the expression level of the spider silk protein, in which the peptide was repeated 48 times, depending on the binding site length by SDS-PAGE and Bradford assay (Biorad, USA), it could be confirmed that the expression was the highest at an 18-nt binding site length.

The spider silk expression ability of the *eno-*knockdown strain constructed using this method was compared with those of the glyA-amplified strain and the RnpA expression-inhibited strain, which are existing strains for increased production of spider silk. As a result, it could be confirmed that the spider silk protein expression level in the eno-knockdown group with an 18-nt binding site length was similar to or slightly higher than those in the existing strains for increased production (FIG. 5).

Thereby, knockdown experiments on additional targets were conducted in order to obtain a strain, which exhibits increased production compared to existing systems, through additional engineering based on *eno* knockdown.

**[Table 2] Vector sequence encoding sRNA target binding region of eno**

| SEQ ID NO. | Name | Sequence |
|---|---|---|
| 25 | Eno24 | ATGTCCAAAATCGTAAAAATCATC |
| 26 | Eno23 | ATGTCCAAAAT CGTAAAAAT CAT |
| 27 | Eno22 | ATGTCCAAAATCGTAAAAATCA |
| 28 | Eno21 | ATGTCCAAAATCGTAAAAATC |
| 29 | Eno20 | ATGTCCAAAATCGTAAAAAT |
| 30 | Eno19 | ATGTCCAAAATCGTAAAAA |
| 16 | Eno18 | ATGTCCAAAATCGTAAAA |
| 31 | Eno17 | ATGTCCAAAATCGTAAA |
| 32 | Eno16 | ATGTCCAAAATCGTAA |
| 33 | Eno15 | ATGTCCAAAATCGTA |

**[Table 3] Primers used in cloning**

| SEQ ID NO. | Name | Sequence |
|---|---|---|
| 34 | ColA_sRNA_Univ ersal F | GCAACCATTATCACCGCCAGAGGTAAAA |
| 35 | eno_24_R | GATGATTTTTACGATTTTGGACATTTTCTGTTGGGCCATTGCA TT |
| 36 | eno_23_R | ATGATTTTTACGATTTTGGACATTTTCTGTTGGGCCATTGCAT T |
| 37 | eno_22_R | TGATTTTTACGATTTTGGACATTTTCTGTTGGGCCATTGCATT |
| 38 | eno_21_R | GATTTTTACGATTTTGGACATTTTCTGTTGGGCCATTGCATT |
| 39 | eno_20_R | ATTTTTACGATTTTGGACATTTTCTGTTGGGCCATTGCATT |
| 40 | eno_19_R | TTTTTACGATTTTGGACATTTTCTGTTGGGCCATTGCATT |
| 41 | eno_18_R | TTTTACGATTTTGGACATTTTCTGTTGGGCCATTGCATT |
| 42 | eno_17_R | TTTACGATTTTGGACATTTTCTGTTGGGCCATTGCATT |
| 43 | eno_16_R | TTACGATTTTGGACATTTTCTGTTGGGCCATTGCATT |
| 44 | eno_15_R | TTACGATTTTGGACATTTTCTGTTGGGCCATTGCATT |

### Example 4. Selection of additional target genes

Based on the pACYC184-Cm vector, synthetic regulatory sRNAs for knocking down additional target genes were constructed using the pR promoter, micC scaffold, and T1/TE terminator (Table 5). Since there was an essential gene among the additional targets, the present inventors performed screening based on a 20-nt binding site length and tried to optimize the length of the target binding site for the group with the highest expression level.

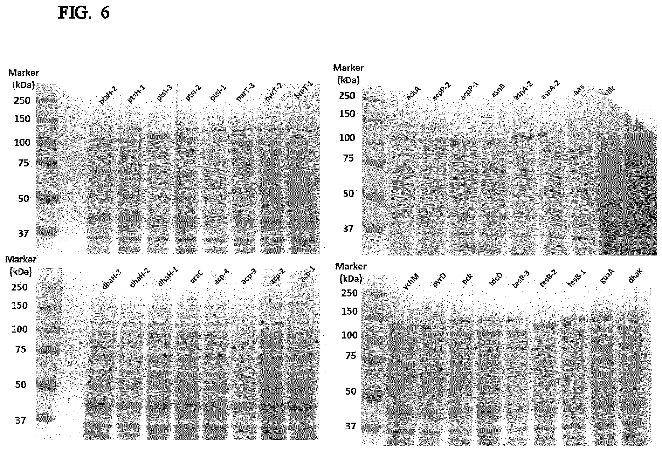

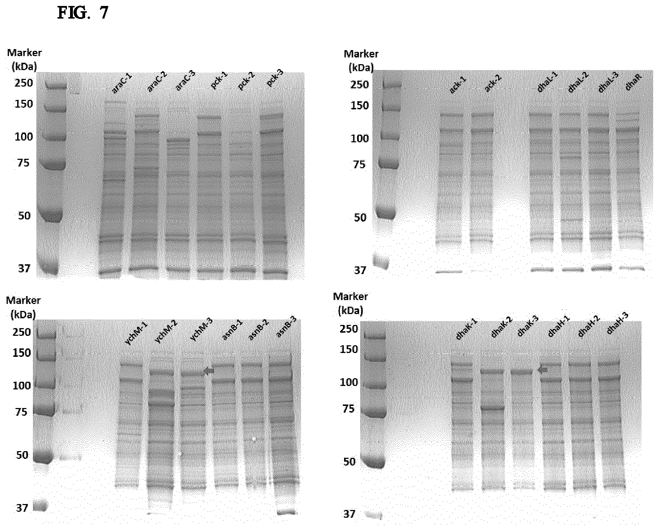

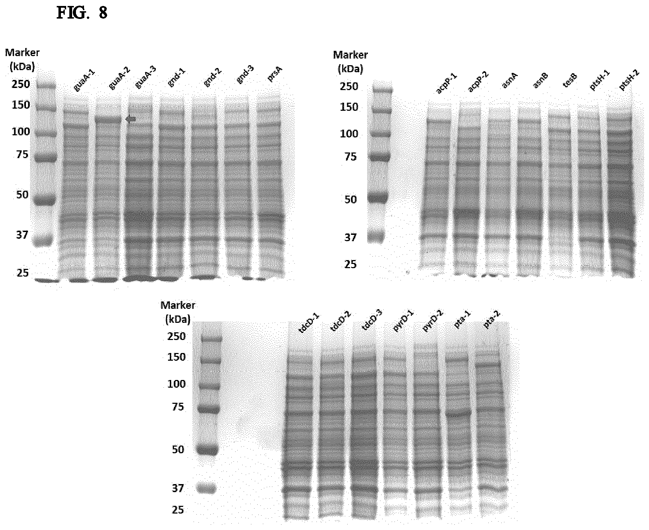

About 30 gene pairs were selected from about 20 reaction pairs selected through MOMA simulation. As a result of analyzing the expression level of the spider silk protein, in which the peptide was repeated 48 times, by SDS-PAGE and Bradford assay (Biorad, USA), it was confirmed that the spider silk protein expression level in the eight gene groups (*pstI, asnA, ychM, tesB, dhaK, guaA, pck,* and *purT*) after first screening (n=3) was similar to or higher than that in the existing strain (FIGS. 6 to 8).

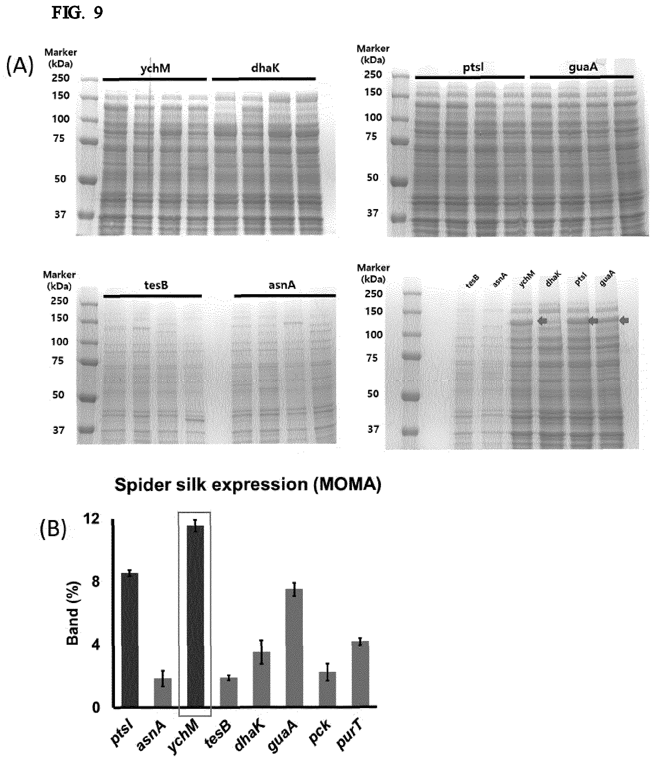

As a result of reconfirming the spider silk protein expression in the above 8 gene groups and performing second screening, it could be confirmed that the spider silk protein expression in the *ychM* and *pstI* groups was much higher than that in the existing strain (FIG. 9). Thereamong, the best growth and protein expression were confirmed in the *ychM* group, which was then selected as the final group, and optimization of knockdown efficiency for that group was performed (Table 4) .

At this time, to calculate binding energy, the UNAFOLD (Unified Nucleic Acid Folding) program, which calculates the binding energy depending on the length and sequence information of nucleotides, was used (Markham, Nicholas R., and Michael Zuker. Bioinformatics. Humana Press, 2008).

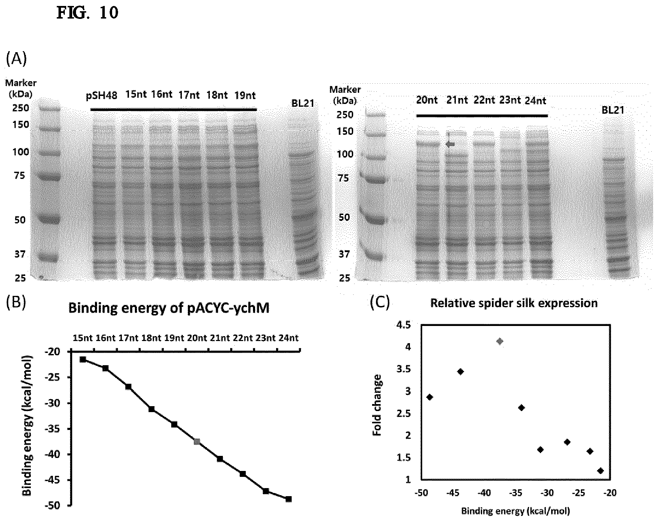

Thereby, it could be confirmed that, as the length of the binding site increased, the binding energy decreased and the knockdown effect increased. Finally, as a result of analyzing the expression level of the spider silk protein, in which the peptide was repeated 48 times, depending on the binding site length by SDS-PAGE and Bradford assay (Biorad, USA), it could be confirmed that expression was the highest at a 20-nt *ychM* binding site length (FIG. 10).

**[Table 4] Vector sequence encoding sRNA target binding region of ychM**

| SEQ ID NO. | Name | Sequence |
|---|---|---|
| 45 | ychM24 | GTGAACAAAATATTTTCCTCACAT |
| 46 | ychM23 | GTGAACAAAATATTTTCCTCACA |
| 47 | ychM22 | GTGAACAAAATATTTTCCTCAC |
| 48 | ychM21 | GTGAACAAAATATTTTCCTCA |
| 19 | ychM20 | GTGAACAAAATATTTTCCTC |
| 49 | ychM19 | GTGAACAAAATATTTTCCT |
| 50 | ychM18 | GTGAACAAAATATTTTCC |
| 51 | ychM17 | GTGAACAAAATATTTTC |
| 52 | ychM16 | GTGAACAAAATATTTT |
| 53 | ychM15 | GTGAACAAAATATTT |

**[Table 5] Primers used in cloning**

| SEQ ID NO. | Name | Sequence |
|---|---|---|
| 54 | Universal_F | gcaaccattatcaccgccagaggtaaaa |
| 55 | asnB_R | ACGCCAAAAATTGAACACATTTTCTGTTGGGCCATTGCATT |
| 56 | asnA_R | GCAATGTAAGCGGTTTTCATTTTCTGTTGGGCCATTGCATT |
| 57 | pck_R | AAACCATTGTTAACGCGCATTTTCTGTTGGGCCATTGCATT |
| 58 | ackA_R | AGTACTAACTTACTCGACATTTTCTGTTGGGCCATTGCATT |
| 59 | pta_R | AGCATAATAATACGGGACACTTTCTGTTGGGCCATTGCATT |
| 60 | ptsl_R | GCTAAAATGCCTGAAATCATTTTCTGTTGGGCCATTGCATT |
| 61 | aas_R | CGAAAAAAGCTAAAAAGCATTTTCTGTTGGGCCATTGCATT |
| 62 | gnd_R | CCGATCTGTTGCTTGGACATTTTCTGTTGGGCCATTGCATT |
| 63 | prsA_R | AAAAGCTTCATATCAGGCACTTTCTGTTGGGCCATTGCATT |
| 64 | tdcD_R | ACAACCGGAAATTCATTCATTTTCTGTTGGGCCATTGCATT |
| 65 | tesB_R | TTTTTTAGCGCCTGACTCATTTTCTGTTGGGCCATTGCATT |
| 66 | acpP_R | CGTTCTTCGATAGTGCTCATTTTCTGTTGGGCCATTGCATT |
| 67 | araC_R | TCATTTTGCGCTTCAGCCATTTTCTGTTGGGCCATTGCATT |
| 68 | dhaH_R | ACTATGACCAGGTTTACCATTTTCTGTTGGGCCATTGCATT |
| 69 | dhaK_R | TCATTGATCAATTTTTTCATTTTCTGTTGGGCCATTGCATT |
| 70 | dhaL_R | TGAGTTCTGCTCAGTGACATTTTCTGTTGGGCCATTGCATT |
| 71 | dhaR_R | TTGTTAAAAGCGCCACTCATTTTCTGTTGGGCCATTGCATT |
| 72 | fsaA_R | GTATCCAGATACAGTTCCATTTTCTGTTGGGCCATTGCATT |
| 73 | fasB_R | GTGTCCAGATACAGTTCCATTTTCTGTTGGGCCATTGCATT |
| 74 | guaA_R | TTATGAATGTTTTCCGTCATTTTCTGTTGGGCCATTGCATT |
| 75 | ptsH_R | GTAACTTCTTGCTGGAACATTTTCTGTTGGGCCATTGCATT |
| 76 | purT_R | GCAGTGCCTAATAACGTCATTTTCTGTTGGGCCATTGCATT |
| 77 | pyrD_R | CGAACGAAGGGGTAGTACATTTTCTGTTGGGCCATTGCATT |
| 78 | ychM_24_R | ATGTGAGGAAAATATTTTGTTCACTTTCTGTTGGGCCATTGCATT |
| 79 | ychM_23_R | TGTGAGGAAAATATTTTGTTCACTTTCTGTTGGGCCATTGCATT |
| 80 | ychM_22_R | GTGAGGAAAATATTTTGTTCACTTTCTGTTGGGCCATTGCATT |
| 81 | ychM_21_R | TGAGGAAAATATTTTGTTCACTTTCTGTTGGGCCATTGCATT |
| 82 | ychM_20_R | GAGGAAAATATTTTGTTCACTTTCTGTTGGGCCATTGCATT |
| 83 | ychM_19_R | AGGAAAATATTTTGTTCACTTTCTGTTGGGCCATTGCATT |
| 84 | ychM_18_R | GGAAAATATTTTGTTCACTTTCTGTTGGGCCATTGCATT |
| 85 | ychM_17_R | GAAAATATTTTGTTCACTTTCTGTTGGGCCATTGCATT |
| 86 | ychM_16_R | AAAATATTTTGTTCACTTTCTGTTGGGCCATTGCATT |
| 87 | ychM_15_R | AAATATTTTGTTCACTTTCTGTTGGGCCATTGCATT |

### Example 5. Confirmation of recombinant silk protein production in final strain

Finally, the group in which *eno* and *ychM* among the 30 target pairs selected in the MOMA simulation were knocked down was selected as the group with the best expression.

As a result of confirming the production of spider silk in the selected strain through 5-L fed-batch fermentation, it was confirmed that the strain showed the highest expression 6 hours after induction with 1 mM IPTG at an OD₆₀₀ of 120 in culture at 30°C, and at this time, the 48-mer high-molecular-weight spider silk showed a high expression level corresponding to about 16% in terms of band intensity as determined by SDS-PAGE, which corresponds to about 10.5 g/L as determined by Bradford assay (FIG. 11). This is about 10 times higher than the previous highest titer (Xia, 2009, PNAS).

### Example 6. Final gene selection and analysis of improved strain

After final gene selection, RNA Seq was performed to analyze the improved strain. To this end, for a spider silk protein-producing strain sampled 6 hours after induction with 1 mM IPTG when the OD₆₀₀ reached 0.4 in 50-ml baffled flask culture at 30°C, and the spider silk protein-producing strain with inhibited expression of *eno* and *ychM* genes, which was normalized to an OD₆₀₀ of 4, RNA Seq was performed by Macrogen.

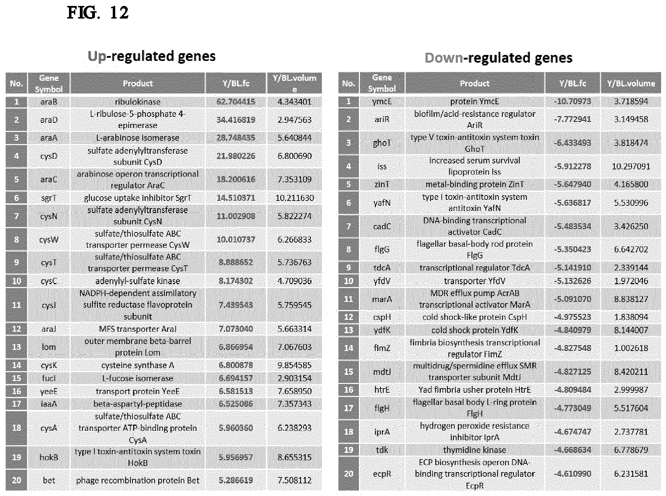

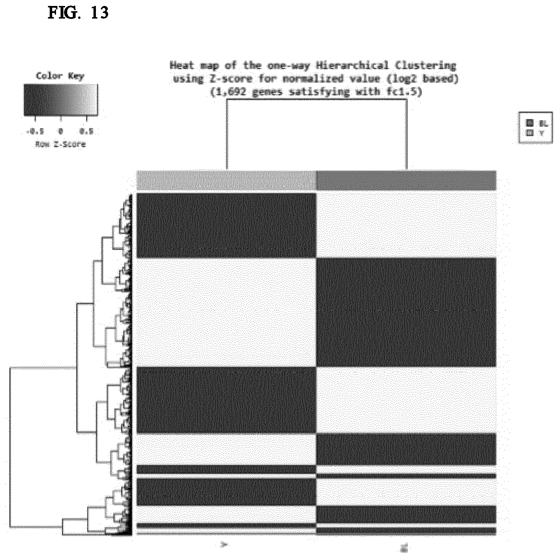

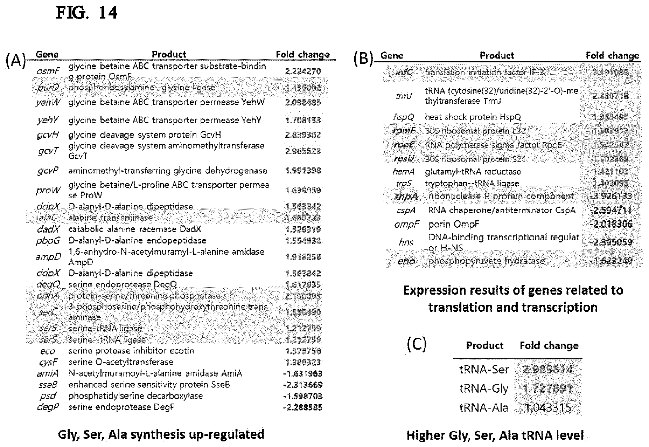

As a result of checking the heatmap for the DEG list using RNA-seq of the strain in which the expression of the selected *eno* gene and *ychM* gene has been inhibited, it could be confirmed that various genotypes were changed compared to those in the existing recombinant silk protein-producing strain (FIGS. 12 and 13). Thereamong, changes in expression of glycine-, alanine-, and serine-related genes, changes in expression of genes related to transcription and translation, and amplification of glycine, alanine, and serine tRNA could be confirmed (FIG. 14) .

In addition, as a result of comparing the metabolic pathway in the wild-type strain with the metabolic pathway of the strain in which *eno* and *ychM* were knocked down, it was confirmed that, in the strain in which *eno* and *ychM* were knocked down, the process of converting glyceraldehyde 3-phosphate to pyruvate was inhibited, and the protein production pathways that form major structures such as serine, glycine, and alanine were further activated. In addition, the action of the fumarate and succinate exporters was inhibited, which inhibited the progress of the TCA cycle, and similarly, it is predicted that the protein production pathways that form major structures such as serine, glycine, and alanine are further activated (FIG. 11A).

### Example 7. Confirmation of mass production of recombinant silk protein by improved strain

In order to use spider silk protein industrially, the present inventors tried to optimize the isolation and purification process, which is one of the major challenges in recombinant protein production. In order to isolate protein from a large amount of fermentation broth relatively easily and with high efficiency, protein precipitation using salting out was performed. Usually, this method is used for protein concentration, but produced high molecular weight spider silk has a very large size of 147 kDa and there are not many proteins with a size larger than that in *E. coli.* Accordingly, it was expected that if the precipitation method was optimized using these characteristics, the desired target could be purified easily and with high efficiency.

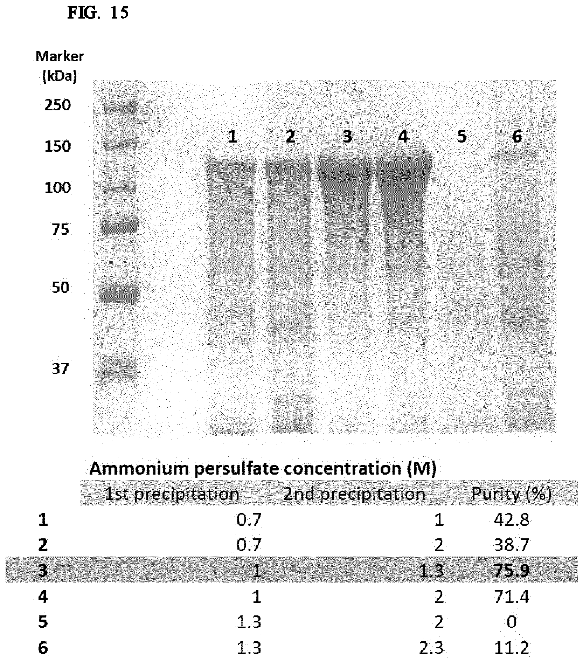

Therefore, as a result of separating and purifying the fermentation broth using various ammonium persulfate concentrations in first purification and second precipitation, it was confirmed that, when 1.0M ammonium persulfate in the first purification and 1.3M ammonium persulfate in the second purification were added, the purification efficiency was the highest with a purity of about 75% (FIG. 15).

In addition, as a result of analyzing the purified sample after microfiltration through a 50-kDa membrane filter (FIG. 16), it was confirmed that other proteins except for the very large spider silk protein were filtered out, and thus a higher purity corresponding to about 80% or more could be obtained. The same result (a purity of about 85%) was confirmed even in mass separation and purification conducted under the same conditions after mass fermentation at 30 L (FIG. 18).

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The recombinant silk protein derived from the recombinant strain according to the present invention has a high molecular weight similar to that of dragline silk protein produced by spiders, and may be mass-produced, and thus it has properties similar to or better than those of natural silk protein when manufactured into fibers. Accordingly, the recombinant silk protein may be advantageously used in various industrial fields such as bioengineering and/or pharmaceutical fields where spider silk fibers are expected to be applied.

### Sequence Listing Free Text

Electronic file attached

## Claims

1. A recombinant microorganism having enhanced ability to produce spider silk protein, obtained by inhibiting expression of *eno* gene in a microorganism having ability to produce recombinant silk protein.

2. The recombinant microorganism according to claim 1, wherein the inhibiting the expression of the *eno* gene is performed by at least one method selected from the group consisting of a method of introducing a sequence encoding a synthetic sRNA that inhibits the expression of the gene, a method of modifying a promoter, a method of introducing antisense RNA, and a method of introducing siRNA.

3. The recombinant microorganism according to claim 1, wherein expression of at least one additional gene selected from the group consisting of *gnd, ybcF, araC, purT, tdcD, ackA, pta, fsaA, fsaB, ptsH, ptsI, dhal, dhaK, pyrD, guaA, prsA, tesB, aas, acpP, ychM, cysZ, asnA, asnB, acpP, plsB, fadB, sucC, fldA, fade, sucA, sucB,* and *pykA* has been inhibited.

4. The recombinant microorganism according to claim 3, wherein the expression of the at least one additional gene has been inhibited by at least one method selected from the group consisting of a method of introducing a sequence encoding a synthetic sRNA that inhibits the expression of the at least one additional gene, a method of modifying a promoter, a method of introducing antisense RNA, and a method of introducing siRNA.

5. The recombinant microorganism according to claim 3, wherein the at least one additional gene is *pstI, asnA, ychM, tesB, dhaK, guaA, pck* or *purT.*

6. The recombinant microorganism according to claim 1, wherein the microorganism having the ability to produce recombinant silk protein is one into which a gene encoding a recombinant silk protein in which a peptide having the amino acid sequence of SEQ ID NO: 1 is repeated 1 to 160 times, or a recombinant vector containing the gene, and a recombinant vector containing a nucleotide sequence encoding tRNA, have been introduced.

7. The recombinant microorganism according to claim 2 or 4, wherein the synthetic sRNA comprises: an Hfq binding site derived from sRNA of any one of MicC, SgrS and MicF; and a region forming a complementary bond with an mRNA of the *eno* gene.

8. The recombinant microorganism according to claim 1, wherein the microorganism is selected from the group consisting of *E. coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium.*

9. The recombinant microorganism according to claim 6, wherein the peptide is a repeating peptide unit constituting a dragline silk protein.

10. A method for producing recombinant silk protein, the method comprising steps of: (a) producing recombinant silk protein by culturing the recombinant microorganism according to any one of claims 1 to 9; and (b) recovering the produced recombinant silk protein.

11. The method according to claim 10, wherein step (b) comprises a salting-out step.
